**Europäisches Patentamt**

(19) **European Patent Office**

**Office européen des brevets**

(11) Publication number : **0 424 069 B1**

(12) # EUROPEAN PATENT SPECIFICATION

(45) Date of publication of patent specification : **29.12.93 Bulletin 93/52**

(51) Int. Cl.⁵ : **B01J 23/80,** C07C 29/141, C07C 29/145

(21) Application number : **90311264.7**

(22) Date of filing : **15.10.90**

(54) **Hydrogenation catalys and process of using said catalyst.**

(30) Priority : **17.10.89 US 422624**

(43) Date of publication of application : **24.04.91 Bulletin 91/17**

(45) Publication of the grant of the patent : **29.12.93 Bulletin 93/52**

(84) Designated Contracting States : **AT BE CH DE DK ES FR GB GR IT LI LU NL SE**

(56) References cited :
**EP-A- 0 152 314**
**DE-A- 3 717 111**
**US-A- 3 388 972**

(73) Proprietor : **ENGELHARD CORPORATION 101 Wood Avenue Iselin, New Jersey 08830-0770 (US)**

(72) Inventor : **Thakur, Deepak S. 7070 Longview Drive Solon, OH 44139 (US)** Inventor : **Sullivan, Thomas J. 16246 Essex Drive Strongsville, OH 44136 (US)** Inventor : **Roberts, Brian D. 32287 Hamilton Drive Solon, OH 44139 (US)** Inventor : **Vlchek, Anita L. 5304 Richards Drive Mentor, OH 44060 (US)**

(74) Representative : **Fisher, Adrian John et al CARPMAELS & RANSFORD 43 Bloomsbury Square London WC1A 2RA (GB)**

## Description

Technical Field

This invention relates to catalysts which are particularly useful as hydrogenation catalysts, and more particularly, as catalysts for hydrogenating aldehydes, ketones, carboxylic acids and carboxylic esters The invention also relates to a method of preparing said catalysts and to the use of the catalysts in hydrogenation reactions.

Background of the Invention

In one embodiment, the present invention relates to catalysts which are useful in hydrogenation reactions and which comprise the oxides of copper, zinc and aluminum. The preparation of various copper-containing catalysts and the use of such catalysts in various reactions has been described previously. Such reactions include hydrogenation reactions, the synthesis of methanol and higher alcohols from synthesis gas,. The copper-containing catalysts also may contain other metal oxides including chromium oxide, zinc oxide, titanium oxide, zirconium oxide, iron oxide, alumina, silica, etc., and mixtures of one or more of said oxides.

The hydrogenation of carboxylic acids and carboxylic esters to alcohols is known in the art, and various methods and catalysts have been suggested for effecting the hydrogenation reaction. For example, the ester may be reduced with lithium aluminum hydride or sodium and alcohol. A commonly practiced method involves the use of a copper-chromite-based hydrogenation catalyst. While copper chromite catalysts are commercially available and successful, the disposal of the spent copper chromite catalyst is a problem since chromium is present in the spent catalyst, and chromium is a highly toxic material which is subject to stringent EPA disposal regulations. Because of the stringent regulations, the cost of the manufacture, use and disposal of copper chromite catalysts has, increased. It is anticipated that in the future, more stringent EPA regulations and escalating disposal costs will adversely affect the economics of using a copper-chromite-based catalyst.

U.S. Patent 2,091,800 describes a copper chromite/barium catalyst which is used in a process for hydrogenating esters at a temperature in the range of 200°C to 400°C by passing the acid and its esters over the hydrogenation catalyst. Other patents describing various types of copper chromite catalysts used in acid and ester hydrogenation processes include U.S. Patent Nos. 2,121,367; 2,782,243; 3,173,959; and 3,267,157.

U.S. Patent 3,894,054 describes the production of tetrahydrofuran by catalytic hydrogenation and dehydration of maleic anhydride using a catalyst composition which comprises a mixture obtained by calcining a silica-alumina catalyst and a copper-chromium-zinc catalyst. U.S. Patent 3,971,735 describes the preparation of methanol from syngas with a catalyst comprised of copper, zinc, aluminum and boron. The hydrogenation of esters to alcohols by contacting the ester with hydrogen and a catalyst comprising cobalt, zinc and copper under catalytic hydrogenation conditions is described in U.S. Patent 4,113,662. U.S. Patent 4,279,781 describes a methanol synthesis catalyst which comprises the oxides of copper and zinc and a minor amount of a thermal stabilizing metal oxide such as alumina. The copper to zinc metal-weight ratio is in the range of from 2:1 to 3.5:1. Catalysts comprising copper, cobalt, a metal selected from chromium, iron, vanadium or manganese, a rare earth metal and a small amount of an alkali or alkaline earth metal are described in U.S. Patent 4,291,126. Optionally, the catalyst may contain zinc and/or a noble metal and/or a binder selected from alumina, magnesia and cements. U.S. Patent 4,440,668 describes a three-component oxide catalyst based on copper, a metal from Group VIA, VIIA or VIIIA, and a metal of Group IVA or VA. The preferred catalyst is based on copper, cobalt and zirconium with the first two components being formed by co-precipitation in the presence of the oxide of the third component. Another multi-component catalytic system is described in U.S. Patent 4,513,100 which comprises zinc, chromium, copper, one or more alkaline metals and possibly one or more metals chosen from molybdenum, manganese, lanthanum, cerium, aluminum, titanium and vanadium.

U.S. Patent 4,535,071 describes a catalyst for methanol synthesis from syngas which comprises as catalytically active substances, copper oxide and zinc oxide and as a thermal stabilizing substance, aluminum oxide. Optimum yields of methanol are obtained when the atomic ratio of copper to zinc is between 2.8 and 3.8. Five-component catalyst compositions are described in U.S. Patent 4,551,444 and the essential components are copper, an iron group component, a component of elements 23-26, an alkaline metal compound and a precious metal compound. Catalysts comprising copper oxide and zinc oxide in a ratio of 8:1 to 1:1 are described in U.S. Patent 4,588,848 as being useful in synthesizing neoalcohols from neoacids. U.S. Patent 4,598,061 describes a catalyst for synthesis of methanol and alcohol mixtures from synthesis gas using a catalyst which contains, as an oxide precursor, copper oxide and zinc oxide; aluminum oxide as a thermal stabilizing substance; and at least one alkali metal compound. Catalysts comprising copper and cobalt, and optionally aluminum and/or zinc and/or sodium are utilized in U.S. Patent 4,675,343 for preparing primary aliphatic alcohols from hydrogen and carbon oxides. The catalysts contain a minimum of 3% cobalt. Catalysts containing the oxides of copper, zinc and alumina are described in U.S. Patent 4,704,480 as being useful in the production of aliphatic ketones and an optional consecutive production of the corresponding carbinols. More specifically, catalysts comprising the oxides of copper, zinc and alumina are utilized in Examples 1 and 11 of the patent and a catalyst comprising the oxides of copper and alumina is utilized in Example 12. Copper-zinc catalysts also are described in U.S. Patent 4,808,562, and the catalysts may contain alumina.

U.K. Patent 1,436,773 also describes copper oxide, zinc oxide catalysts obtained by coprecipitation which are suitable for use in the synthesis of methanol from synthesis gas. The ratio of copper to zinc in the catalyst is from 1:1 to 8:1, and the catalyst may contain a thermal stabilizer such as alumina. Japanese Patent 62-53740 apparently describes catalysts derived from the nitrates of copper, zinc, manganese/magnesium and aluminium.

German Offenlegungschrift 2,613,226 describes a continuous preparation of fatty alcohols by catalytic hydrogenation of relatively high molecular weight fatty acids and esters formed with low-molecular weight monohydric alcohols. The process utilizes hydrogen and a catalyst. The catalysts disclosed in that specification include copper chromite or copper-zinc-chromite and copper-zinc catalysts with or without known carrier substances.

Although many copper-containing catalysts have been described in the prior art, there continues to be a need for catalysts which are useful particularly in the hydrogenation of aldehydes, acids and esters, including diesters. It is also desirable to prepare catalysts useful in hydrogenation reactions which can be carried out in either a fixed bed or a fluidized bed reactor.

Summary of the Invention

In one aspect, the present invention provides a catalyst in powdered form which comprises a major amount of the oxides of copper and zinc, and a minor amount of aluminium oxide wherein the pore volume of pores of said catalyst having a diameter of greater than 80Å (i.e. 80 ångströms or 8 nm) is at least 80% of the total pore volume.

In another aspect, the present invention provides a process for hydrogenating an aldehyde, ketone, carboxylic acid or carboxylic acid ester to an alcohol comprising contacting the aldehyde, ketone, acid or ester with hydrogen and a catalyst under catalytic hydrogenation conditions, characterised by using a catalyst comprising a major amount of the oxides of copper and zinc, and a minor amount of aluminium oxide wherein the pore volume of pores of said catalyst having a diameter of greater than 80Å (8nm) is at least 80% of the total pore volume.

Catalysts of the invention are useful in both fixed bed and slurry phase hydrogenation reactions.

Description of the Drawings

Fig. 1 is a graph illustrating the methyl ester hydrogenation activity of the catalyst prepared in Examples 1-8 and 12 as well as control examples C-1 to C-5 and a commercial catalyst with respect to percent conversion.

Fig. 2 is a graph illustrating the relative methyl ester hydrogenolysis activity (with respect to saponification value) of the catalyst of Example 1 compared with the activity of several catalysts containing other metal combinations.

Description of the Preferred Embodiments

In one preferred embodiment of the present invention, the catalyst in powder form comprises a major amount of the oxides of copper and zinc and a minor amount of aluminium oxide wherein the pore volume of pores having a diameter of greater than 80 A (8 nm) is at least about 85% of the total pore volume. All references to pore diameters and pore volumes in the specification and claims are based upon measurements utilizing mercury porosimetry. A typical method is described by R. Anderson, Experimental Methods in Catalytic Research, Academic Press, New York, 1968. The pore volumes are determined utilizing the powder forms of the catalysts in their oxide forms. That is, the pore diameters reported herein are obtained for the powder catalyst after calcination, but prior to any reduction of the oxide. Those skilled in the art often refer to the catalyst containing the metal oxides as the "oxide" or "oxidic precurser" form of the catalyst.

The powdered catalysts of the present invention which contain-a major amount of the oxides of copper and zinc, and a minor amount of aluminum oxide, also may be characterized as having an average surface area of at least about 70 square meters per gram and more generally from 70 to 200 square meters per gram. The powdered catalysts also may be characterized as having an average particle diameter of from 8 to 28 microns.

In another embodiment, the catalysts of the present invention may be characterized as having a copper to zinc atomic ratio of from 0.2 to 5.5, or from 0.2 to 3.0. In other embodiments, the atomic ratio of copper to zinc may be less than about 1.

In a further embodiment, the present invention relates to a catalyst wherein the atomic ratio of copper to zinc is less than about 0.85. More particularly, the atomic ratio of copper to zinc is from 0.25 to 0.85.

The amount of aluminum oxide contained in the catalyst of this invention may be varied over a wide range although the catalyst will generally contain from 2% to 40% by weight of aluminum oxide, and more often contains from 5% to 30% by weight of aluminum oxide. The catalyst of the invention also may be characterized by an atomic ratio of copper and zinc to aluminum of less than about 15.

Various procedures can be utilized to prepare the catalysts of the present invention. For example, the mixture of water-soluble salts of copper and zinc may be mixed together followed by the addition of alumina and thereafter the base to precipitate the metal components. Alternatively, the copper and zinc salts may be mixed together followed by the addition of an aqueous alkaline solution such as a solution of soda ash, in the presence of hydrated alumina powder while maintaining the pH at 7 to 8 as the precipitate is formed and recovered.

A preferred process for preparing the hydrogenation catalyst comprising the oxides of copper, zinc and aluminum, comprises the steps of

(A) preparing a first aqueous solution containing at least one water-soluble copper salt and at least one water-soluble zinc salt;

(B) preparing a second solution containing at least one water-soluble basic aluminum salt and at least one alkaline precipitating agent;

(C) mixing the first and second solutions whereby an insoluble solid is formed;

(D) recovering the insoluble solid; and

(E) calcining the recovered solid.

The catalyst prepared in accordance with the above method may be characterized as having an atomic ratio of copper to zinc of from 0.2 to 5.5. Such catalysts are prepared by maintaining the atomic ratio of copper to zinc in the first aqueous solution within the indicated range. In another embodiment, the atomic ratio of copper to zinc in the first aqueous solution is from 0.2 to 3.0, and in other embodiments the ratio may be less than about 1, or less than about 0.85. The amount of alumina contained in the catalyst recovered from the calcination of the removed solid in step (E) may contain from 2% to 40% by weight of aluminum oxide, and more generally, from 5% to 30% by weight of aluminum oxide.

The first and second solutions described above may be mixed in any manner or order. Thus, the first solution can be added to the second solution, or the second solution can be added to the first solution, or a mixture of the two solutions can be obtained by simultaneously mixing the two solutions such as by simultaneously adding the two solutions to a vessel. It is desirable that the mixing of the first and second solutions in step (C) be conducted at a pH above about 5.5, and more generally above about 7.0. When the two solutions are mixed simultaneously, the pH of the resulting mixture can be controlled by varying the rate of addition of the second solution which contains an alkaline material. As the rate of addition of the second solution increases, the pH of the resulting mixture increases.

The water-soluble copper and zinc salts utilized to form the first solution are copper and zinc salts such as the nitrates, acetates, sulfates, chlorides, etc. It is presently preferred, however, to use the nitrates of copper and zinc in the formation of the first solution. Any water-soluble aluminum salt can be utilized to prepare the second solution, and the aluminum salt generally is a basic aluminum salt such as sodium aluminate. Alumina gels also can be utilized.

The second solution also contains at least one alkaline material which may be a soluble base such as sodium hydroxide, sodium carbonate, ammonium hydroxide, ammonium carbonate, etc., and mixtures thereof. The amount of alkaline material included in the second solution may be varied over a wide range, and the amount of alkaline materials should be sufficient to provide an alkaline solution which, when added to the first solution will result in a mixture having the desired pH. The pH of the mixture obtained by mixing the first and second solutions should be within the range of from 5.5 to 9.0 and more preferably is at least 7, and most preferably at least about 7.5. As noted above, the pH of the mixture can be maintained as desired by adjusting the relative addition rates of the two solutions. Additionally, the mixture obtained from the first and second solutions should be maintained at a temperature of from 50-80°C. A precipitate is formed and recovered by techniques well known in the art such as by filtration, centrifugation, etc. The recovered precipitate preferably is washed with water to remove impurities, dried by heating to a temperature of up to about 150°C, and finally calcined. The recovered precipitate is calcined at a temperature in the range of from 475°C to 700°C for a period of 30 to 120 minutes. Generally, calcination at a temperature of 500°C for 30 minutes is sufficient.

The catalysts which are obtained by the above-described co-precipitation procedure generally are characterized as having an average particle size of from 8 to 28 microns, and the particles have an average surface area of at least about 70 square meters per gram. In one embodiment, the surface area will range from 70 to 200 square meters per gram. The pore volume of pores in said catalysts having a diameter of greater than about 80Å (8 nm) is at least about 80% of the total pore volume, and more often, more than 85% of the total pore volume. In another embodiment, more than 75% of the pores have a diameter in excess of 120 Å.

The following examples illustrate the above-described preferred embodiment for preparing the hydrogenation catalyst of the present invention. Unless otherwise indicated in the examples and elsewhere in the specification and claims, all parts and percentages are by weight, temperatures are in degrees Centigrade, and pressures are at or near atmospheric.

Example 1

A first solution is prepared from 710 parts of an aqueous copper nitrate solution containing 16.3% copper, 620 parts of zinc nitrate tetrahydrate and 1600 parts water. A second solution is prepared by dissolving 330 parts sodium aluminate (23% Al) and 350 parts soda ash in 2500 parts water. The two solutions are mixed in a reaction vessel that contains 5000 parts water at 70°C. The reaction temperature is maintained at 70°C (±5°C) and the pH is maintained at about 7.5. A precipitate is formed and recovered by filtration. The recovered precipitate is washed with water, dried at 125°C and finally calcined at 500°C (±10°C) for 30 minutes. The physical and chemical characteristics of the calcined product are given in Table I.

Example 2

A first solution is prepared from 467 parts of an aqueous copper nitrate solution containing 16.3% copper, 933 parts of an aqueous zinc nitrate solution containing 16.5% zinc and 200 parts water. A second solution is prepared by dissolving 35 parts sodium aluminate (23% Al), 126 parts of an aqueous solution of sodium hydroxide (50% NaOH) and 413 parts soda ash in 1200 parts water. The two solutions are mixed in a reaction vessel that contains 5000 parts water at 70°C. The reaction temperature is maintained at 70°C (±5°C), and the pH is maintained at about 7.5. A precipitate is formed and recovered by filtration. It is then washed with water, dried at 125°C and finally calcined at 500°C (±10°C) for 30 minutes. The physical and chemical characteristics of the calcined product are given in Table I.

Example 3

A first solution is prepared from 351 parts of an aqueous copper nitrate solution containing 16.3% copper, 1050 parts of an aqueous zinc nitrate solution containing 16.5% zinc and 220 parts water. A second solution is prepared by dissolving 35 parts sodium aluminate (23% Al), 126 parts of an aqueous solution of sodium hydroxide (50% NaOH) and 413 parts soda ash in 1200 parts water. The two solutions are mixed in a reaction vessel that contains 5000 parts water at 70°C. The reaction temperature is maintained at 70°C (±5°C), and the pH is maintained at about 7.5. A precipitate is formed and recovered by filtration. It is then washed with water, dried at 125°C and calcined at 500°C for 30 minutes. The physical and chemical characteristics of the calcined product are given in Table I.

Example 4

A first solution is prepared from 622 parts of an aqueous copper nitrate solution containing 16.3% copper, 778 parts of an aqueous zinc nitrate solution containing 16.5% zinc and 200 parts water. A second solution is prepared by dissolving 35 parts sodium aluminate (23% Al), 86 parts of an aqueous solution of sodium hydroxide (50% NaOH) and 413 parts soda ash in 1237 parts water. The two solutions are mixed in a reaction vessel that contains 5000 parts water at 70°C, and the pH is maintained at about 7.5. A precipitate is formed and recovered by filtration. It is then washed with water, dried at 125°C and calcined at 500°C for 30 minutes. The physical and chemical characteristics of the calcined product are given in Table I.

Example 5

A first solution is prepared from 350 parts of an aqueous copper nitrate solution containing 16.3% copper, 1050 parts of an aqueous zinc nitrate solution containing 16.5% zinc and 200 parts water. A second solution is prepared by dissolving 330 parts sodium aluminate (23% Al), 21 parts of an aqueous solution of sodium hydroxide (50% NaOH) and 320 parts soda ash in 1601 parts water. The two solutions are mixed in a reaction vessel that contains 5000 parts water at 70°C. The reaction temperature is maintained at 70°C, and the pH is maintained at about 7.5. A precipitate is formed and recovered by filtration. It is then washed with water, dried at 125°C and calcined at 500°C for 30 minutes. The physical and chemical characteristics of the calcined product are given in Table I.

Example 6

A first solution is prepared from 710 parts of an aqueous copper nitrate solution containing 16.5% zinc and 1452 aqueous zinc nitrate solution containing 16.5% zinc and 1452 parts water. A second solution is prepared by dissolving 330 parts sodium aluminate (23% Al), 30 parts of an aqueous solution of sodium hydroxide (50% NaOH) and 350 parts soda ash in 2252 parts water. The two solutions are mixed in a reaction vessel that contains 5000 parts water at 70°C. The reaction temperature is maintained at 70°C, and the pH is maintained at about 7.5. A precipitate is formed and recovered by filtration. It is then washed with water, dried at 125°C and calcined at 500°C for 30 minutes. The physical and chemical characteristics of the calcined product are given in Table I.

Example 7

A first solution is prepared from 324 parts of an aqueous copper nitrate solution containing 16.3% copper, 1291 parts of an aqueous zinc nitrate solution containing 16.5% zinc and 1489 parts water. A second solution is prepared by dissolving 330 parts sodium aluminate (23% Al), 10 parts of an aqueous solution of sodium hydroxide (50% NaOH) and 350 parts soda ash in 2212 parts water. The two solutions are mixed in a reaction vessel that contains 5000 parts water at 70°C, and the pH is maintained at about 7.5. A precipitate is formed and recovered by filtration. It is then washed with water, dried at 125°C and calcined at 500°C for 30 minutes. The physical and chemical characteristics of the calcined product are given in Table IA.

Example 8

A first solution is prepared from 710 parts of an aqueous copper nitrate solution containing 16.3% copper, 899 parts of an aqueous zinc nitrate solution containing 16.5% zinc and 1414 parts water. A second solution is prepared by mixing 286 parts of aqueous solution of sodium aluminate (13% Al), 21 parts of an aqueous solution of sodium hydroxide (50% NaOH) and 350 parts soda ash in 1100 parts water. The two solutions are mixed in a reaction vessel that contains 5000 parts water at 70°C. The reaction temperature is maintained at 70°C, and the pH is maintained at about 7.5. A precipitate is formed and recovered by filtration. It is then washed with water, dried at 125°C and calcined at 600°C for 45 minutes. The physical and chemical characteristics of the calcined product are given in Table IA.

Example 9

A first solution is prepared from 1000 parts of an aqueous nitrate solution containing 16.3% copper, 335 parts of an aqeuous zinc nitrate solution containing 16.5% zinc and 1600 parts water. A second solution is prepared by dissolving 113 parts sodium aluminate (23% Al) and 490 parts soda ash in 2500 parts water. The two solutions are mixed in a reaction vessel that contains 5000 parts water at 70°C. The reaction temperature is maintained at 70°C (±5°C), and the pH of the mixture is maintained at 7.5. A precipitate is formed and recovered by filtration. It is then washed with water, dried at 125°C and finally calcined at 500°C (±10°C) for 75 minutes. The physical and chemical characteristics of the calcined product are shown in Table IA.

Example 10

A first solution is prepared from 1000 parts of an aqueous copper nitrate solution containing 16.4% copper, 253 parts of an aqueous zinc nitrate solution containing 16.5% zinc and 1600 parts water. A second solution is prepared by dissolving 37 parts sodium aluminate (23% Al) and 450 parts soda ash in 2300 parts water. The two solutions are mixed in a reaction vessel that contains 5000 parts water at 70°C. The reaction temperature is maintained at 70°C (±5°C), and the pH of the mixture is maintained at about 7.5. A precipitate is formed and recovered by filtration. It is then washed. with water, dried at 125°C and finally calcined at 480°C (±10°C) for 50 minutes. The physical and chemical characteristics of the calcined product are shown in Table IA.

Example 11

A first solution is prepared from 1000 parts of an aqueous copper nitrate solution containing 16.3% copper, 200 parts of an aqueous zinc nitrate solution containing 16.5% zinc and 200 parts water. A second solution is prepared by dissolving 35 parts sodium aluminate (23% Al), 126 parts of an aqueous sodium hydroxide solution (50% NaOH), and 413 parts soda ash in 1000 parts water. The two solutions are mixed in a reaction vessel that contains 5000 parts water at 70°C. The reaction temperature is maintained at 70°C (±5°C), and the pH of the mixture is maintained at about 7.5. A precipitate is formed and recovered by filtration. It is then washed with water, dried at 125°C and finally calcined at 500°C (±10°C) for 30 minutes. The physical and chemical characteristics of the calcined product are shown in Table IA.

Example 12

A first solution is prepared from 701 parts of an aqueous copper nitrate solution containing 16.3% copper, 702 parts of an aqueous zinc nitrate solution containing 16.5% zinc and 208 parts water. A second solution is prepared by dissolving 415 parts soda ash in 1000 parts water. The two solutions are mixed in a reaction vessel that contains 5000 parts water in which 100 parts of a hydrated alumina powder (Kaiser Versal® 850) is dispersed. The reaction temperature is maintained at 70°C (±5°C), and the pH is maintained at about 7.5. A precipitate is formed and recovered by filtration. It is then washed with water, dried at 125°C and calcined at 500°C (±10°C) for 30 minutes. The physical and chemical characteristics of the calcined product are given in Table IA.

## TABLE I

| | Example | | | | | |
|---|---|---|---|---|---|---|
| | 1 | 2 | 3 | 4 | 5 | 6 |
| Cu/Zn Molar Ratio | 0.82 | 0.52 | 0.36 | 0.84 | 0.35 | 0.81 |
| (Cu+Zn)/Al Ratio | 1.22 | 12.3 | 8.79 | 11.89 | 1.46 | 2.06 |
| Surf. Area ($M^2$/g) | 173 | 90.9 | 89.1 | 85.3 | 165 | 143 |
| He Density (g/cc) | 4.32 | 5.16 | 4.62 | 4.77 | 3.73 | 3.96 |
| Hg Density @18.5 PSI (127.6 kPa) | 0.44 | 0.50 | 0.50 | 0.49 | 0.57 | 0.49 |
| Pore Diameter (Å)* | | | | | | |
| Up to 60 | 10.2 | 3.4 | 5.2 | 0 | 6.2 | 6.8 |
| Up to 80 | 11.4 | 4.4 | 6 | 0 | 9.5 | 7.7 |
| Up to 100 | 12.7 | 5.6 | 7.6 | 0 | 12.9 | 8.9 |
| Up to 120 | 13.5 | 7.3 | 9.6 | 0 | 15.2 | 10.1 |
| Up to 350 | 15.6 | 19.4 | 20.9 | 18.7 | 20.6 | 18.1 |
| Up to 700 | 16.9 | 23.7 | 27.5 | 25.5 | 22.7 | 24.5 |
| Up to 1000 | 17.9 | 25.4 | 30.2 | 28 | 24.1 | 27.5 |
| Up to 10000 | 48.3 | 43.1 | 48.6 | 45.8 | 42.2 | 47.1 |
| Average Particle Size (μm) | 22 | 15.8 | 15.2 | 15.1 | 13.4 | 18.7 |
| Chemical Analysis at 500°C (% wt.) | | | | | | |
| Copper | 23.8 | 24.8 | 18.2 | 33.3 | 13.6 | 22.93 |
| Zinc | 30.0 | 48.8 | 51.5 | 40.6 | 39.6 | 29.00 |
| Aluminum | 14.6 | 2.50 | 3.30 | 2.60 | 15.2 | 14.40 |

---

\*    Cumulative percentage.    1 Å = 0.1 nm.

## TABLE IA

|  | Example | | | | | |
|---|---|---|---|---|---|---|
|  | 7 | 8 | 9 | 10 | 11 | 12 |
| Cu/Zn Molar Ratio | 0.26 | 0.82 | 1.94 | 4.08 | 5.16 | 1.07 |
| (Cu+Zn)/Al Ratio | 1.75 | 2.29 | 2.07 | 10.4 | 12.3 | 2.33 |
| Surf. Area ($M^2$/g) | 179 | ---- | 79.9 | 70.1 | 69.0 | 48.9 |
| He Density (g/cc) | 4.37 | 4.39 | 4.92 | 5.10 | 5.20 | 4.00 |
| Hg Density@18.5 PSI (127.6 kPa) | 0.47 | 0.41 | 0.51 | 0.65 | 0.78 | 1.17 |
| Pore Diameter (Å )* | | | | | | |
| Up to 60 | 2.8 | 6.8 | 17 | 5 | 3.4 | 13.3 |
| Up to 80 | 8.6 | 8.7 | 17 | 5.2 | 4.2 | 14.8 |
| Up to 100 | 15.6 | 9.8 | 17 | 6.3 | 4.9 | 16.3 |
| Up to 120 | 20.2 | 10.5 | 17.3 | 8.3 | 6 | 17.9 |
| Up to 350 | 32.2 | 14.3 | 25.3 | 26.1 | 31.7 | 52 |
| Up to 700 | 35.3 | 17.4 | 33.7 | 34.8 | 48.7 | 71.8 |
| Up to 1000 | 36.2 | 19.3 | 40.3 | 39.2 | 55.7 | 75.7 |
| Up to 10000 | 43.5 | 36 | 72.9 | 49.5 | 84.1 | 83.8 |
| Average Particle Size (μm) | 17.1 | 17.4 | 23 | 22.3 | 16.9 | 57.5 |
| Chemical Analysis at 500°C (% wt.) | | | | | | |
| Copper | 10.96 | 25.6 | 44.5 | 58.7 | 61.9 | 28.1 |
| Zinc | 43.90 | 32.0 | 23.6 | 14.8 | 12.2 | 27.0 |
| Aluminum | 13.05 | 7.1 | 2.96 | 2.6 | 15.2 | 9.9 |

\* Cumulative percentage.    1 Å = 0.1 nm.

The following examples C-1 to C-6 illustrate the preparation of control catalysts which are not within the present invention but are presented herein for comparison purposes.

Example C-1

(Cu/Zn)

A first solution is prepared from 1000 parts of an aqueous copper nitrate solution containing 16.3% copper, 1017 parts of an aqueous zinc nitrate solution containing 16.5% zinc and 689 parts water. A second solution is prepared from 374 parts of soda ash in 1012 parts water. The two solutions are mixed in a reaction vessel that contains 2000 parts water at 45°C. The pH is maintained at about 7.5, and a precipitate is formed and recovered by filtration. It is then washed with water, dried at 125°C and calcined at 500°C for 30 minutes. The physical and chemical characteristics of the calcined product are given in Table IB.

Example C-2

(Cu/Al)

A first solution is prepared from 1010 parts of an aqueous copper nitrate solution containing 16.3% copper and 1640 parts water. A second solution is prepared from 374 parts of soda ash in 1012 parts water. The two solutions are mixed in a reaction vessel that contains 2000 parts water at 45°C. The pk is maintained at about 7.5, and a precipitate is formed and recovered by filtration. It is then washed with water, dried at 125°C and calcined at 500°C for 30 minutes. The physical and chemical characteristics of the calcined product are given in Table IB.

Example C-3

(Zn/Al)

A first solution is prepared from 1015 parts of an aqueous zinc nitrate solution containing 16.5% zinc and 1745 parts water. A second solution is prepared by dissolving 342 parts sodium aluminate (23% Al) and 250 parts soda ash in 2210 parts water. The two solutions are mixed in a reaction vessel that contains 5000 parts water at 70°C. The reaction temperature is maintained at 70°C and the pH is maintained at about 7.5. A precipitate is formed and recovered by filtration. It is then washed with water, dried at 125°C and calcined at 500°C for 30 minutes. The physical and chemical characteristics of the calcined product are given in Table IB.

Example C-4

(Cu/Zn/Al/Co)

A first solution is prepared from 500 parts of an aqueous copper nitrate solution containing 16.3% copper, 254 parts of an aqueous zinc nitrate solution containing 16.5% zinc, and 17 parts of cobalt nitrate hexahydrate. A second solution is prepared by mixing 115 parts of sodium aluminate (23% Al) and 250 parts soda ash in 2485 parts water. The two solutions are mixed in a reaction vessel that contains 2140 parts water at 70°C. The reaction temperature is maintained at about 70°C, and the pH is maintained at about 7.5. A precipitate is formed and recovered by filtration. It is then washed with water, dried at 125°C and calcined at 500°C for 30 minutes. The physical and chemical characteristics of the calcined product are given in Table IB.

Example C-5

(Cu/Zn/Co)

A first solution is prepared from 501 parts of an aqueous copper nitrate solution containing 16.3% copper, 255 parts of an aqueous zinc nitrate solution containing 16.5% zinc, and 34 parts of cobalt nitrate hexahydrate. A second solution is prepared from 365 parts soda ash in 2487 parts water. The two solutions are mixed in a reaction vessel that contains 2140 parts water at 70°C. The reaction temperature is maintained at about 70°C, and the pH is maintained at about 7.5. A precipitate is formed and recovered by filtration. It is then washed with water, dried at 125°C and calcined at 500°C for 30 minutes. The physical and chemical characteristics of the calcined product are given in Table IB.

Example C-6

(Cu/Cr/Mn)

A first solution made up of 600 parts of a copper nitrate solution (16.3% Cu), 31.5 parts of a manganese nitrate solution (15.5% Mn) and 175 parts chromic acid ($CrO_3$) and a second solution made up of 362 parts concentrated ammonium hydroxide and 132 parts water are added to a reaction vessel, containing 1500 parts water at 50°C, to form a precipitate. The precipitate is collected by filtration and washed with water. After drying at 150°C for 12 hours, the material is calcined at 420°C for 30 minutes. The physical and chemical characteristics of the calcined product are given in Table IB.

TABLE IB

| | Example | | | | | |
| | C-1 | C-2 | C-3 | C-4 | C-5 | C-6 |
|---|---|---|---|---|---|---|
| Cu/Zn Molar Ratio | 1.01 | 1011 | 0.00 | 2.11 | 2.14 | ---- |
| (Cu+Zn)/Al Ratio | ---- | 1.25 | 0.69 | 2.28 | ---- | ---- |
| Surf. Area ($M^2/g$) | 16.6 | 82.4 | 158 | 98.3 | ---- | 60 |
| He Density (g/cc) | 5.06 | 3.56 | 4.09 | 4.01 | 5.81 | 4.9 |
| Hg Density@18.5 PSI (127.6 kPa) | 1.08 | 0.47 | 0.46 | 0.38 | 1.21 | 1.6 |
| Pore Diameter (Å)* | | | | | | |
| Up to 60 | 6.2 | 0 | 1.1 | 2 | 7.6 | |
| Up to 80 | 6.2 | 0.3 | 3.3 | 2 | 7.6 | |
| Up to 100 | 6.2 | 0.9 | 7.4 | 2 | 7.6 | |
| Up to 120 | 6.2 | 1.6 | 10.8 | 2 | 7.6 | |
| Up to 350 | 6.9 | 13.6 | 20.2 | 7 | 29.9 | |
| Up to 700 | 32.2 | 25.1 | 23.8 | 14.6 | 43.5 | |
| Up to 1000 | 42.5 | 32 | 25.7 | 19.1 | 48.3 | |
| Up to 10000 | 75.4 | 87.1 | 54.3 | 53.2 | 76.5 | |
| Average Particle Size (μm) | 19.5 | 17.2 | 18.3 | 22.3 | 12.6 | 22 |
| Chemical Analysis at 500°C (% wt.) | | | | | | |
| Copper | 34.5 | 49.1 | 0.0 | 39.2 | 51.0 | 35.7 |
| Zinc | 35.0 | 0.05 | 29.4 | 19.1 | 24.6 | Cr=31.7 |
| Aluminum | ---- | 16.7 | 17.6 | 11.4 | ---- | ---- |
| Other | ---- | ---- | ---- | Co=3.1 | Co=4.2 | Mn=3.7 |

* Cumulative percentage. 1 Å = 0.1 nm.

The catalysts of the present invention which contain copper, zinc and alumina have been found to be particularly useful for hydrogenating aldehydes, ketones, carboxylic acids and carboxylic esters to alcohols. Generally, these catalysts do not contain chromium or cobalt. In one embodiment, the atomic ratio of copper to zinc in the catalysts is from about 0.5 to 5.5, and in one preferred embodiment, the ratio is less than about 0.85, and the catalyst is also free of boron. In another preferred embodiment, the hydrogenation catalyst is characterized by an atomic ratio of copper to zinc of less than about 0.85, the catalyst is free of chromium, cobalt and boron, and the catalyst is prepared by the preferred process as illustrated in Examples 1-8.

The catalysts of the present invention which are prepared as powders may be utilized in slurry-(liquid-) phase hydrogenation processes. Alternatively, the powders can be processed into shapes such as pellets and used in fixed bed reactors. In one embodiment, carboxylic acids and carboxylic esters can be converted to alcohols in excellent yields. A wide variety of acids, particularly esters of carboxylic acids can be treated with the catalyst of the present invention to produce alcohols. The esters may be monoesters or diesters. Among the acids which may be hydrogenated to the corresponding alcohols without isolating the ester include stearic acids and caproic acids. Esters derived from the alcohols of higher molecular weight carboxylic acids are hydrogenated more rapidly and at lower temperatures than the esters derived from the lighter alcohols. Examples of esters which may be hydrogenated with the catalyst of the present invention include the methyl ester of coconut fatty acid, methyl stearate, methyl oleate, ethyl laurate, ethyl myristate, the diethyl ester of ethyl malonic acid, diethyl succinate, di-n-butyl glutarate, diethyl sebacate. As noted, the esters are converted to alcohols, and examples of such conversions include: ethyl laurate to lauryl alcohol; ethyl myristate to myristyl alcohol; ethyl valerate to n-amyl alcohol; methyl caproate to n-hexyl alcohol.

Examples of aldehydes which may be hydrogenated with the catalyst of the present invention include: butyraldehyde, furfural, 2-ethyl-hexanal, dodecanal, and tetradecanal. Examples of ketones include acetone, and acetophenone.

The hydrogenation reactions which are conducted in the presence of the catalyst of the present invention are carried out at temperatures of from 250°C to 350°C and at pressures of from 1500 psi to 4500 psi (10342 to 31026 kPa).

In one preferred embodiment, the hydrogenation reaction is conducted in a batch or continuous ebullated bed reactor. In this process, the catalyst powder particles are slurried with the aldehyde, ketone, carboxylic acid or carboxylic ester to be reduced, and there is intimate contact between the catalyst and the liquid. When one of the preferred catalysts of the present invention wherein the atomic ratio of copper to zinc is less than about 0.85, and the catalyst is prepared by the preferred coprecipitation procedure such as illustrated in Examples 1-8, is used in a batch ebullated reactor, high yields of alcohols are obtained in shorter times, and the slurry, upon completion of the hydrogenation reaction is easily filtered.

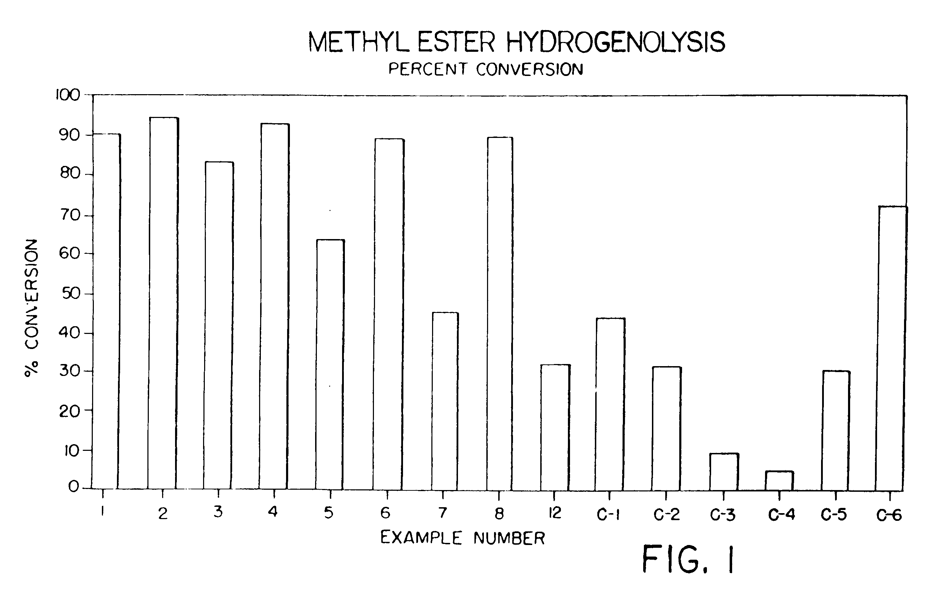

The effectiveness of the catalyst of Examples 1-8 and 12 of the present invention as hydrogenation catalysts is illustrated by utilizing the catalysts to hydrogenate coconut methyl-ester at 280°C, 3000 psig (20684 kPa) hydrogen and 1.8 weight percent catalyst loading, and measuring the percent conversion to the alcohol after the hydrogenation reaction has been conducted for 60 minutes. The results are summarized in the graph of Fig. 1. Also contained in Fig. 1 are the results of the hydrogenation reaction conducted with other catalysts which are not within the scope of the present invention which have been identified above as Examples C-1 to C-6.

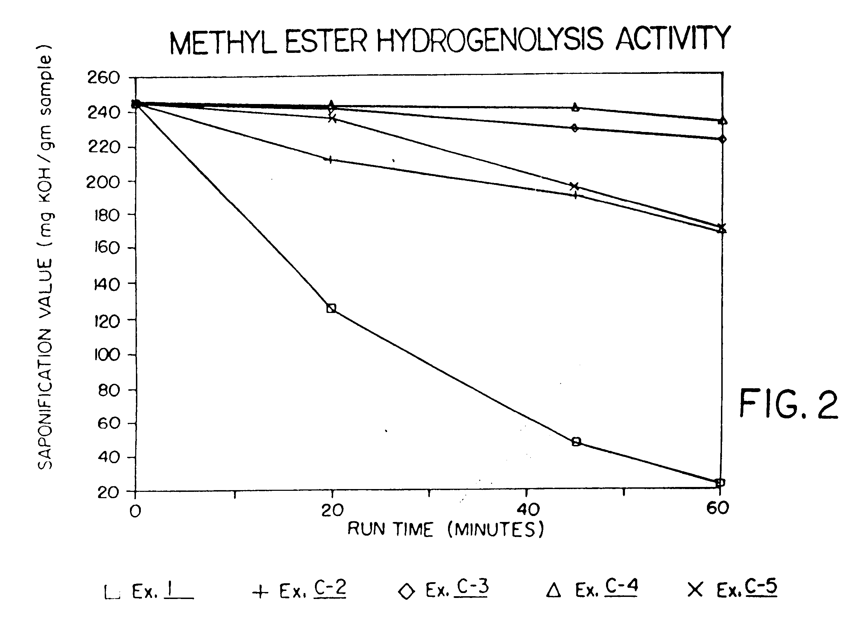

The hydrogenation activity of the catalyst of Example 1 also has been compared to the catalysts of Examples C-2 through C-5 by carrying out hydrogenation reactions of coconut methyl ester at 280°C, 3000 psig (20684 kPa) of hydrogen and 1.8 weight percent catalyst loading. The effectiveness of the catalyst is determined by measuring the saponification value for each of the samples pursuant to A.O.C.S. official method Cd 3-25, revised April, 1966, reapproved 1973. Under these specific test conditions, saponification values of below about 50 indicate good hydrogenation activity. The results of this test are shown in Fig. 2, and the results demonstrate the improved hydrogenation activity of the catalyst of Example 1 when compared to the catalysts of Examples C-2 to C-5.

The process for hydrogenating a diester, namely, hydrodimethoxyterephthalate is illustrated as follows. The fixed-bed reaction is carried out using a pelletized version of a catalyst similar to that described in Example 9. The reaction is performed in an autoclave type reactor in which the catalyst is contained in a perforated, closed-ended tube known as a thimble and is in contact with the reaction medium. The reaction is carried out at 250-275°C, and 2000-5000 psig (13790 - 34474 kPa) of hydrogen. Catalytic activity is determined by measuring the percent conversion by gas chromatography. The catalyst of the present invention is at least 50% more active than commercial copper chromite catalysts.

## Claims

1.  A catalyst in powdered form comprising a major amount of the oxides of copper and zinc, and a minor amount of aluminium oxide wherein the pore volume of the pores in said catalyst having a diameter of greater than 80Å (8nm) is at least 80% of the total pore volume.

2.  The catalyst of claim 1 wherein the pore volume of the pores having a diameter of greater than 80Å (8nm) is at least 85% of the total pore volume.

3.  The catalyst of claim 1 or 2 wherein the powders have a surface area of at least 70 square meters per gram.

4.  The catalyst of claim 1, 2 or 3, wherein the powders have an average particle diameter of from 8 to 28 microns (μm).

5. The catalyst of any of claims 1 to 4 wherein the atomic ratio of copper to zinc is from 0.2 to 5.5.

6. The catalyst of claim 5 wherein the atomic ratio of copper to zinc is from 0.2 to 3.0.

7. The catalyst of any of claims 1 to 6 wherein the atomic ratio of copper to zinc is less than 1.

8. The catalyst of claim 7 wherein the copper to zinc atomic ratio is less than 0.85.

9. The catalyst of claim 7 wherein the atomic ratio of copper to zinc is from 0.25 to 0.85.

10. The catalyst of any of claims 1 to 9 containing from 0.5 to 40% by weight of aluminium oxide.

11. The catalyst of claim 10 containing from 3 to 40% by weight of aluminium oxide.

12. The catalyst of claim 10 containing from 5 to 30% by weight of aluminium oxide.

13. The catalyst of any of claims 1 to 12 wherein the atomic ratio of copper and zinc to aluminium is less than 15.

14. The catalyst of any of claims 1 to 13 which is free of chromium and cobalt.

15. A process for hydrogenating an aldehyde, ketone, carboxylic acid or carboxylic acid ester to an alcohol comprising contacting the aldehyde, ketone, acid or ester with hydrogen and a catalyst under catalytic hydrogenation conditions, characterised by using a catalyst comprising a major amount of the oxides of copper and zinc, and a minor amount of aluminium oxide wherein the pore volume of pores of said catalyst having a diameter of greater than 80Å (8nm) is at least 80% of the total pore volume.

16. The hydrogenation process of claim 15 conducted in the liquid phase.

17. The hydrogenation process of claim 15 or 16 wherein the catalyst is according to any one of claims 2 to 14.


**Patentansprüche**

1. Katalysator in gepulverter Form, umfassend eine Hauptmenge von den Oxiden von Kupfer und Zink, und eine Nebenmenge von Aluminiumoxid, worin das Porenvolumen der Poren in dem Katalysator mit einem Durchmesser, der größer als 80Å (8 nm) ist, mindestens 80% des gesamten Porenvolumens beträgt.

2. Katalysator nach Anspruch 1, worin das Porenvolumen der Poren mit einem Durchmesser, der größer als 80Å (8 nm) ist, mindestens 85% des gesamten Porenvolumens beträgt.

3. Katalysator nach Anspruch 1 oder 2, worin die Pulver eine Oberfläche von mindestens 70 m$^2$ pro Gramm haben.

4. Katalysator nach Anspruch 1, 2 oder 3, worin die Pulver einen mittleren Partikeldurchmesser von 8 bis 28 Mikrometer ($\mu$m) haben.

5. Katalysator nach einem der Ansprüche 1 bis 4, worin das Atomverhältnis von Kupfer zu Zink 0,2 bis 5,5 beträgt.

6. Katalysator nach Anspruch 5, worin das Atomverhältnis von Kupfer zu Zink 0,2 bis 3,0 beträgt.

7. Katalysator nach einem der Ansprüche 1 bis 6, worin das Atomverhältnis von Kupfer zu Zink weniger als 1 beträgt.

8. Katalysator nach Anspruch 7, worin das Atomverhältnis von Kupfer zu Zink weniger als 0,85 beträgt.

9. Katalysator nach Anspruch 7, worin das Atomverhältnis von Kupfer zu Zink 0,25 bis 0,85 beträgt.

10. Katalysator nach einem der Ansprüche 1 bis 9, welcher 0,5 bis 40 Gewichtsprozent Aluminiumoxid enthält.

**11.** Katalysator nach Anspruch 10, welcher 3 bis 40 Gewichtsprozent Aluminiumoxid enthält.

**12.** Katalysator nach Anspruch 10, welcher 5 bis 30 Gewichtsprozent Aluminiumoxid enthält.

**13.** Katalysator nach einem der Ansprüche 1 bis 12, worin das Atomverhältnis von Kupfer und Zink zu Aluminium weniger als 15 beträgt.

**14.** Katalysator nach einem der Ansprüche 1 bis 13, welcher frei von Chrom und Kobalt ist.

**15.** Verfahren zum Hydrieren eines Aldehyds, Ketons, einer Carbonsäure oder eines Carbonsäureesters zu einem Alkohol, umfassend das In-Kontakt-Bringen des Aldehyds, Ketons, der Säure oder des Esters mit Wasserstoff und einem Katalysator unter katalytischen Hydrierungsbedingungen, **dadurch gekennzeichnet,** daß ein Katalysator verwendet wird, der eine Hauptmenge der Oxide von Kupfer und Zink und eine Nebenmenge von Aluminiumoxid umfaßt, worin das Porenvolumen von Poren des Katalysators mit einem Durchmesser, der größer als 80 Å (8nm) ist, mindestens 80% des gesamten Porenvolumens beträgt.

**16.** Hydrierungsverfahren nach Anspruch 15, welches in der flüssigen Phase durchgeführt wird.

**17.** Hydrierungsverfahren nach Anspruch 15 oder 16, worin der Katalysator nach einem der Ansprüche 2 bis 14 ist.

**Revendications**

**1.** Catalyseur sous forme de poudre comprenant une quantité majeure des oxydes de cuivre et de zinc, et une quantité mineure d'oxyde d'aluminium, dans lequel le volume de pores des pores dans ledit catalyseur ayant un diamètre supérieur à 80 Å (8 nm) est d'au moins 80% du volume total de pores.

**2.** Catalyseur selon la revendication 1, dans lequel le volume de pores des pores ayant un diamètre supérieur à 80 Å (8 nm) est d'au moins 85% du volume total de pores.

**3.** Catalyseur selon la revendication 1 ou 2, dans lequel les poudres ont une surface spécifique d'au moins 70 m² par gramme.

**4.** Catalyseur selon la revendication 1, 2 ou 3 dans lequel les poudres ont un diamètre de particule moyen de 8 à 28 micromètres ($\mu$m)

**5.** Catalyseur selon l'une quelconque des revendications 1 à 4, dans lequel le rapport atomique du cuivre au zinc est de 0,2 à 5,5.

**6.** Catalyseur selon la revendication 5, dans lequel le rapport atomique du cuivre au zinc est de 0,2 à 3,0.

**7.** Catalyseur selon l'une quelconque des revendications 1 à 6, dans lequel le rapport atomique du cuivre au zinc est inférieur à 1.

**8.** Catalyseur selon la revendication 7, dans lequel le rapport atomique du cuivre au zinc est inférieur à 0,85.

**9.** Catalyseur selon la revendication 7, dans lequel le rapport atomique du cuivre au zinc est de 0,25 à 0,85.

**10.** Catalyseur selon l'une quelconque des revendications 1 à 9 contenant de 0,5 à 40% en poids d'oxyde d'aluminium.

**11.** Catalyseur selon la revendication 10 contenant de 3 à 40% en poids d'oxyde d'aluminium.

**12.** Catalyseur selon la revendication 10 contenant de 5 à 30% en poids d'oxyde d'aluminium.

**13.** Catalyseur selon l'une quelconque des revendications 1 à 12, dans lequel le rapport du cuivre et du zinc à l'aluminium est inférieur à 15.

**14.** Catalyseur selon l'une quelconque des revendications 1 à 13 qui est exempt de chrome et de cobalt.

15. Procédé d'hydrogénation d'un aldéhyde, d'une cétone, d'un acide carboxylique ou d'un ester d'acide carboxylique en un alcool comprenant l'opération consistant à mettre en contact l'aldéhyde, la cétone, l'acide ou l'ester avec de l'hydrogène et un catalyseur dans des conditions d'hydrogénation catalytique, caractérisé par l'utilisation d'un catalyseur comprenant une quantité majeure des oxydes de cuivre et de zinc et une quantité mineure d'oxyde d'aluminium dans lequel le volume de pores des pores dudit catalyseur ayant un diamètre supérieur à 80 Å (8 nm) est d'au moins 80% du volume total de pores.

16. Procédé d'hydrogénation selon la revendication 15, conduite en phase liquide.

17. Procédé d'hydrogénation selon la revendication 15 ou 16, dans lequel le catalyseur est selon l'une quelconques des revendications 2 à 14.

## METHYL ESTER HYDROGENOLYSIS
### PERCENT CONVERSION

FIG. 1

FIG. 2

METHYL ESTER HYDROGENOLYSIS ACTIVITY

SAPONIFICATION VALUE (mg KOH / gm sample)

RUN TIME (MINUTES)

□ Ex. 1    + Ex. C-2    ◇ Ex. C-3    △ Ex. C-4    × Ex. C-5